# EUROPEAN PATENT APPLICATION

(11) **EP 4 356 895 A1**
(43) Date of publication of application: **24.04.2024**
(21) Application number: 23165244.7
(22) Date of filing: 29.03.2023
(51) Int. Cl.: A61K 8/02, A61K 8/06, A61K 8/36, A61K 8/63, A61K 8/68, A61Q 19/00

(54) **LIQUID CRYSTAL COMPOSITION INCLUDING CERAMIDE AND METHOD OF PREPARING THE SAME**

(30) Priority: 20.10.2022 KR 20220135791
(71) Applicant: Croda Korea Limited, Iksan-si, Jeollabuk-do 54588 (KR)
(72) Inventor: HAN, Ji Hye, 16858 Yongin-si (KR); KIM, Sang Chul, 16858 Yongin-si (KR); SUNG, Mi Kyung, 16858 Yongin-si (KR); LIM, So Jung, 16858 Yongin-si (KR); PARK, Seung Won, 16858 Yongin-si (KR)
(74) Representative: de Saint Viance, Isabelle Marie Fanny

(57) **Abstract**

Provided a liquid crystal composition including ceramide, a method of preparing the same, and a cosmetic composition including the same. According to a liquid crystal composition including various types of ceramide of an aspect and a method of preparing the same, the liquid crystal composition not only has high liquid crystal stability that liquid crystal is maintained even after 4 weeks at a high temperature, but also activities of increasing an amount of ceramide, increasing an expression level of aquaporin 3, increasing an amount of filaggrin, increasing an expression level of hyaluronic acid, increasing an expression level of loricrin, increasing an expression level of involucrin, and increasing a thickness of the skin, resulting in effects usefully available for amelioration of the skin conditions.

## Description

### BACKGROUND

### 1. Field

The present disclosure relates to liquid crystal compositions comprising ceramide, methods of preparing the same, and cosmetic compositions including the same.

### 2. Description of the Related Art

Ceramide is mainly produced by hydrolysis of an enzyme called sphingomyelinase in sphingomyelin which is a type of phospholipid constituting cell membranes. As a main polar lipid component of the stratum corneum, which accounts for about 50 % of the skin stratum corneum, ceramide is a component that forms a lipid bilayer with moisture to have anti-moisture and barrier functions.

The surface of human skin is protected by corneocytes that are differentiated keratinocytes, wherein the keratinocytes are differentiated and matured from the basal layer. These corneocytes bind to the skin surface by a double-chained lamellar structure of ceramide excessively present in the stratum corneum, so that the skin retains the elasticity. That is, when the ceramides are arranged side by side in a layered structure, and moisture is filled and maintained between the ceramides, such a layered structure may exhibit a barrier function overall.

In addition, in light of the fact that abnormal ceramide levels (i.e., sphingolipid deficiency) are commonly observed in atopic eczema, dermatitis, and psoriasis, it is confirmed that the relationship between the skin health and sphingolipids is very close.

Research results have already been reported in the academic world that external supplementation of ceramide can restore the lamellar structure of the skin and restore the skin to the normal state. It is known that ceramide not only supplies moisture to the skin, but also has a moisturizing effect that prevents moisture of the skin from escaping to the outside. As these effects of ceramide are known, ceramide is currently widely used as a major component of functional cosmetics, skin and hair moisturizers, and the like.

Meanwhile, a liquid crystal composition including ceramide has a structure similar to a composition of the liver lipid of the biological cell membranes of the skin, and thus a moisturizing film similar to the liver lipid of the skin may be formed, so as to effectively provide moisturizing and protective functions to the skin. However, since the liquid crystal composition cannot include ceramide in a large amount, and the stability of the formed liquid crystal is not sufficient, there are many problems in practical use.

Therefore, regarding the liquid crystal composition including ceramide, a technique for developing a stable liquid crystal formulation while including ceramide in a large amount is needed.

### SUMMARY

Provided is a liquid crystal composition including: a water phase; and an oil phase including a skin lipid, an emulsifier, and/or oil, wherein the skin lipid includes ceramide, cholesterol or a derivative thereof, and/or a fatty acid.

Provided is a method of preparing the liquid crystal composition, the method including: preparing an oil phase by mixing and dissolving a skin lipid that includes ceramide, cholesterol or a derivative thereof, and/or a fatty acid; preparing a water phase; and mixing the oil phase and the water phase.

Provided is a cosmetic composition including the liquid crystal composition.

Provided is a composition of external skin preparation including the liquid crystal composition.

Additional aspects will be set forth in part in the description which follows and, in part, will be apparent from the description, or may be learned by practice of the presented embodiments of the disclosure.

According to an aspect of an embodiment, a liquid crystal composition includes: a water phase; and an oil phase including a skin lipid, an emulsifier, and/or oil, wherein the skin lipid includes ceramide, cholesterol or a derivative thereof, and/or a fatty acid.

The water phase may refer to an ingredient having hydrophilicity to be mixed with water (purified water).

The water phase may include purified water and polyol.

The terms "polyol" and "polyhydric alcohol" as used in the present specification are used interchangeably, and refer to an aliphatic compound having two or more hydroxyl groups (-OH). The polyol may include glycerols, glycols, or a combination thereof. The glycerols may include glycerin, diglycerin, polyglycerin, glyceritol, glycylalcohol, or a combination of two or more thereof. The glycerin may be used interchangeably with other commonly used names, such as glycerol propane-1,2,3-triol, 1,2,3-propenetriol, trihydroxypropane, and the like. The glycols may include propylene glycol, dipropylene glycol, butylene glycol, diethylene glycol, triethylene glycol, hexylene glycol, pentylene glycol, polyethylene glycol, or a combination of two or more thereof. In detail, the water phase may include purified water, glycerin, and 1,2-hexanediol.

In addition, the water phase may include ingredients for imparting viscosity to the liquid crystal composition. When viscosity is imparted to the liquid crystal composition by the ingredients above, particles may be evenly dispersed when preparing particles including ceramide, and thus particles having excellent stability may be prepared. When particles are prepared without forming viscosity in the liquid crystal composition, the particles are not evenly dispersed and separated in the upper layer, and thus problems with particle stability, such as agglomeration of particles, may occur.

In an embodiment, the water phase may be, based on the total weight of the liquid crystal composition, included in an amount in a range of about 60 wt% to about 90 wt%, for example, about 65 wt% to about 90 wt%, about 70 wt% to about 90 wt%, about 71 wt% to about 90 wt%, about 72 wt% to about 90 wt%, about 73 wt% to about 90 wt%, about 74 wt% to about 90 wt%, about 75 wt% to about 90 wt%, about 76 wt% to about 90 wt%, about 77 wt% to about 90 wt%, about 78 wt% to about 90 wt%, about 79 wt% to about 90 wt%, about 80 wt% to about 90 wt%, about 60 wt% to about 85 wt%, about 65 wt% to about 85 wt%, about 70 wt% to about 85 wt%, about 71 wt% to about 85 wt%, about 72 wt% to about 85 wt%, about 73 wt% to about 85 wt%, about 74 wt% to about 85 wt%, about 75 wt% to about 85 wt%, about 76 wt% to about 85 wt%, about 77 wt% to about 85 wt%, about 78 wt% to about 85 wt%, about 79 wt% to about 85 wt%, or about 80 wt% to about 85 wt%.

The oil phase may refer to an ingredient having hydrophobicity not to be mixed with water (purified water).

The oil phase may include a skin lipid, an emulsifier, and/or oil.

The skin lipid may include ceramide, cholesterol or a derivative thereof, and/or a fatty acid.

The ceramide is used interchangeably with N-acylsphingosine, and may include all compounds consisting of sphingosine and fatty acids. Specific examples of the ceramide may include natural ceramide, synthetic ceramide, pseudo ceramide, or a derivative thereof. The natural ceramide is ceramide that exists in nature, and may include ceramide extracted from animals, plants, and microorganisms. The pseudo ceramide is a general term for compounds having a double-chained lamellar structure of the natural ceramide. Examples of the pseudo ceramide may include at least one selected from 1,3-bis(N-2-(hydroxyethyl)palmitoylamino)-2-hydroxy propane, 1,3-bis(N-2-(hydroxyethyl)lauroyl amino)-2-hydroxy propane, 1,3-bis(N-2-(hydroxyethyl)isostearoylamino)-2-hydroxy propane, 1,3-bis(N-2-(hydroxyethyl) stearoylamino)-2-hydroxypropane, and a mixture thereof.

The ceramide may include one selected from the group consisting of Ceramide 1 (Ceramide EOP), Ceramide 2 (Ceramide NS), Ceramide 3 (Ceramide NP), Ceramide 4 (Ceramide EOH), Ceramide 5 (Ceramide AS), Ceramide 6 (Ceramide AP), Ceramide 7 (Ceramide AH), Ceramide 8 (Ceramide NH), Ceramide 9 (Ceramide EOS), Ceramide 10 (Ceramide Nds), Ceramide 11 (Ceramide Ads), Ceramide 12 (Ceramide EOds), and a combination thereof, or may include a combination of at least two, at least three, at least four, at least five, at least six, or at least seven selected from the group. In detail, the ceramide may include a combination of at least two, at least three, or at least four selected from the group consisting of Ceramide 3 (Ceramide NP), Ceramide 6 (Ceramide AP), Ceramide 1 (Ceramide EOP), Ceramide 10 (Ceramide Nds), Ceramide 11 (Ceramide Ads), and Ceramide 12 (Ceramide EOds).

In an embodiment, the ceramide may be hybrid ceramides in which two or more ceramides are mixed. In the hybrid ceramides, a combination of two or more of the above-mentioned ceramides may be used. In addition, the hybrid ceramides may include two or more ceramides at a specific weight ratio. In an embodiment, the ceramide may be hybrid ceramides in which Ceramide 3 (Ceramide NP) and Ceramide 6 (Ceramide AP) are mixed at a weight ratio in a range of about 5:1 to about 1:1. For example, the hybrid ceramides may include Ceramide 3 (Ceramide NP) and Ceramide 6 (Ceramide AP) at a weight ratio in a range of about 4.5 to 1: 1, about 4.0 to 1: 1, about 3.5 to 1: 1, about 3.0 to 1: 1, about 2.5 to 1: 1, about 2.4 to 1:1, about 2.3 to 1:1, about 2.2 to 1:1, about 2.1 to 1:1, about 2.0 to 1:1, about 1.9 to 1:1, about 1.8 to 1:1, about 1.7 to 1:1, about 1.6 to: 1, about 1.5 to 1: 1, about 1.4 to 1: 1, about 1.3 to 1: 1, about 1.2 to 1: 1, or about 1.1 to 1: 1.

In an embodiment, the ceramide may be hybrid ceramides further including Ceramide 1 (Ceramide EOP).

In an embodiment, the ceramide may be hybrid ceramides further include at least one or at least two selected from the group consisting of Ceramide 1 (Ceramide EOP), Ceramide 10 (Ceramide Nds), and Ceramide 11 (Ceramide Ads), in addition to Ceramide 3 (Ceramide NP) and/or Ceramide 6 (Ceramide AP).

In one or more embodiments, the ceramide may not include other ceramides, for example, pseudo ceramides, in addition to ceramides present in the skin. The expression "substantially does not comprise" or "does not comprise" as used in the present specification means that the ingredient, e.g., pseudo ceramide, is included to such an extent not affecting the physical properties, activity, or pharmacological activity of the liquid crystal composition, or is not included at all. For example, the expression "does not comprise" refers to a case where the pseudo ceramide is included in an amount of 0.005 % or less, 0.001 % or less, or 0.0001 % or less with respect to the liquid crystal composition or cosmetic composition, or may include a case where the pseudo ceramide is not included at all.

The cholesterol or a derivative thereof is an ingredient that has been commonly used in the art to stabilize ceramide in the liquid crystal composition, and types thereof are not particularly limited. For example, the cholesterol or a derivative thereof may be selected from cholesterol, cholestanol, coprostanol, campesterol, stigmasterol, sitosterol, ergosterol, cerevisterol, thymosterol, dihydrocholesterol, cerebrosterol, lanosterol, dihydrolanosterol, agnosterol, dihydroagnosterol, phytosterol, and the like. These may be used alone or in combination of two or more. The cholesterol or a derivative thereof may be included, based on the total weight of the liquid crystal composition, in an amount in a range of about 0.05 wt% to about 5 wt%, about 0.05 wt% to about 4 wt%, about 0.05 wt% to about 3 wt%, about 0.08 wt% to about 5 wt%, about 0.08 wt% to about 4 wt%, about 0.08 wt% to about 3 wt%, about 0.1 wt% to about 5 wt%, about 0.1 wt% to about 4 wt%, about 0.1 wt% to about 3 wt%, about 0.1 wt% to about 2 wt%, about 0.1 wt% to about 1.5 wt%, or about 0.1 wt% to about 1.2 wt%.

The fatty acid may be selected from the group consisting of oleic acid, linoleic acid, behenic acid, arachidonic acid, stearic acid, palmitic acid, myristic acid, and lauric acid, but is not limited thereto. In detail, the fatty acid may be stearic acid. The fatty acid may be included, based on the total weight of the liquid crystal composition, in an amount in a range of about 0.05 wt% to about 5 wt%, about 0.05 wt% to about 4 wt%, about 0.05 wt% to about 3 wt%, about 0.08 wt% to about 5 wt%, about 0.08 wt% to about 4 wt%, about 0.08 wt% to about 3 wt%, about 0.1 wt% to about 5 wt%, about 0.1 wt% to about 4 wt%, or about 0.1 wt% to about 3 wt%.

In an embodiment, the skin lipid may be included, based on the total weight of the liquid crystal composition, in an amount in a range of about 0.1 wt% to about 20 wt%. For example, the amount may be in a range of about 0.1 wt% to about 15 wt%, about 0.1 wt% to about 10 wt%, about 0.1 wt% to about 8 wt%, about 0.1 wt% to about 6 wt%, about 0.3 wt% to about 20 wt%, about 0.3 wt% to about 15 wt%, about 0.3 wt% to about 10 wt%, about 0.3 wt% to about 6 wt%, about 0.5 wt% to about 20 wt%, about 0.5 wt% to about 15 wt%, about 0.5 wt% to about 10 wt%, about 0.5 wt% to about 8 wt%, about 0.5 wt% to about 6 wt%, about 0.7 wt% to about 20 wt%, about 0.7 wt% to about 15 wt%, about 0.7 wt% to about 10 wt%, or about 0.7 wt% to about 6 wt%.

In an embodiment, the skin lipid may include the ceramide, the cholesterol or a derivative thereof, and the fatty acid at a molar ratio in a range of about 1: 1: 1 to about 1: 1.5: 1.5.

In an embodiment, the skin lipid may include the ceramide, the cholesterol or a derivative thereof, and the fatty acid at a molar ratio in a range of about 1: 0.5 to 2: 0.5 to 2. For example, the skin lipid may include the ceramide, the cholesterol or a derivative thereof, and the fatty acid at a molar ratio in a range of about 1 : 0.5 to 1.9 : 0.5 to 1.9, about 1 : 0.5 to 1.8 : 0.5 to 1.8, about 1 : 0.5 to 1.7 : 0.5 to 1.7, about 1 : 0.5 to 1.6 : 0.5 to 1.6, about 1 : 0.5 to 1.5 : 0.5 to 1.5, about 1 : 0.7 to 2 : 0.7 to 2, about 1 : 0.7 to 1.9 : 0.7 to 1.9, about 1 : 0.7 to 1.8 : 0.7 to 1.8, about 1 : 0.7 to 1.7 : 0.7 to 1.7, about 1 : 0.7 to 1.6 : 0.7 to 1.6, about 1 : 0.7 to 1.5 : 0.7 to 1.5, about 1 : 1 to 2 : 1 to 2, about 1 : 1 to 1.9 : 1 to 1.9, about 1 : 1 to 1.8 : 1 to 1.8, about 1 : 1 to 1.7 : 1 to 1.7, about 1 : 1 to 1.6 : 1 to 1.6, or about 1 : 1 to 1.5 : 1 to 1.5.

In an embodiment, the liquid crystal composition may further include glucosyl ceramide. The glucosyl ceramide may be included, based on the total weight of the liquid crystal composition, in an amount in a range of about 0.001 wt% to about 2 wt%, about 0.001 wt% to about 1 wt%, about 0.005 wt% to about 2 wt%, about 0.005 wt% to about 1 wt%, about 0.01 wt% to about 1 wt%, about 0.005 wt% to about 0.5 wt%, or about 0.001 wt% to about 0.5 wt%.

In an embodiment, the liquid crystal composition may include, as a sphingoid base, one selected from the group consisting of sphingosine, phytosphingosine, tetraacetylphytosphingosine, and spinganine. The sphingoid/sphingoid base may be included, based on the total weight of the liquid crystal composition, in an amount in a range of about 0.1 wt% to about 5 wt%. For example, the amount may be in a range of about 0.1 wt% to about 4 wt%, about 0.1 wt% to about 3 wt%, about 0.2 wt% to about 5 wt%, about 0.2 wt% to about 4 wt%, or about 0.2 wt% to about 2 wt%.

The emulsifier may be a nonionic surfactant. The emulsifier may include a combination of two or more selected from the group consisting of glyceryl monostearate, glyceryl laurate, cetyl alcohol, hydrogenated lecithin, sorbitan sesquioleate, sorbitan stearate, sorbitan isostearate, glycol stearate, and glycol distearate. In an embodiment, the nonionic surfactant may include a combination of glyceryl monostearate, cetyl alcohol, and hydrogenated lecithin.

The emulsifier may have low hydrophilic-lipophile balance (HLB). In an embodiment, the HLB of the emulsifier may be in a range of about 3 to 12, for example, about 3 to about 11, about 3 to about 10, about 3 to about 8, about 3 to about 6, or about 3 to about 5. In detail, the HBL of glyceryl stearate may be in a range of about 3.7 to about 3.9, and the HBL of hydrogenated lecithin may be in a range of about 4 to about 9.

In an embodiment, the emulsifier may be included, based on the total weight of the liquid crystal composition, in an amount in a range of about 1 wt% to about 20 wt%. For example, the amount may be in a range of about 1 wt% to about 19 wt%, about 1 wt% to about 18 wt%, about 1 wt% to about 17 wt%, about 1 wt% to about 16 wt%, about 1 wt% to about 15 wt%, about 1 wt% to about 14 wt%, about 1 wt% to about 13 wt%, about 1 wt% to about 12 wt%, about 1 wt% to about 11 wt%, about 1 wt% to about 10 wt%, about 1 wt% to about 9 wt%, about 3 wt% to about 20 wt%, about 3 wt% to about 19 wt%, about 3 wt% to about 18 wt%, about 3 wt% to about 17 wt%, about 3 wt% to about 16 wt%, about 3 wt% to about 15 wt%, about 3 wt% to about 14 wt%, about 3 wt% to about 13 wt%, about 3 wt% to about 12 wt%, about 3 wt% to about 11 wt%, about 3 wt% to about 10 wt%, about 3 wt% to about 9 wt%, about 3 wt% to about 8 wt%, about 5 wt% to about 20 wt%, about 5 wt% to about 19 wt%, about 5 wt% to about 18 wt%, about 5 wt% to about 17 wt%, about 5 wt% to about 16 wt%, about 5 wt% to about 15 wt%, about 5 wt% to about 14 wt%, about 5 wt% to about 13 wt%, about 5 wt% to about 12 wt%, about 5 wt% to about 11 wt%, about 5 wt% to about 10 wt%, about 5 wt% to about 9 wt%, or about 5 wt% to about 8 wt%.

As the oil, various types of oil known in the art may be used. The oil may be ester-based oil, hydrocarbon-based oil, silicone oil, vegetable oil, or a mixture thereof. The ester-based oil may include C₁₂-C₁₅ alkyl octanoate, myristyl lactate, cetyl octanoate, isopropyl myristate, octyldodecyl myristate, hexyl laurate, di(C₁₂-C₁₃) alkyl malate, myristyl lactate, octyldodecyl stearoyl stearate, diisostearyl malate, pentaerythrityl tetraisostearate, diglyceryl triisostearate, or cetyl ethylhexanoate. The hydrocarbon-based oil may include hydrogenated PEG-60 castor oil, hydrogenated polyisobutene, or hydrogenated polydecene. The silicon oil may include dimethicone, polydimethylsiloxane (PDMS), phenyl trimethicone, phenyl dimethicone, phenyl(trimethylsiloxy)diphenylsiloxane, diphenyl dimethicone, diphenyl dimethicone, phenyl silicone, such as diphenyl(methyldiphenyl)trisiloxane or (2-phenylethyl)trimethylsiloxysilicate, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, or dodecamethylpentasiloxane. The vegetable oil may include shea butter oil, mango butter oil, cocoa seed butter oil, macadamia nut oil, olive oil, meadowfoam seed oil, refined grape seed oil, peach seed oil, or sunflower seed oil. In addition, the vegetable oil may include hydrogenated vegetable oil. The term "hydrogenated vegetable oil" includes partially hydrogenated vegetable oil, and may refer to vegetable oil that is partially or entirely hydrogenated by a known technique of chemically adding hydrogen gas to vegetable oil in the presence of a catalyst.

In an embodiment, the oil may be included, based on the total weight of the liquid crystal composition, in an amount in a range of about 5 wt% to about 30 wt%. For example, the amount may be in a range of about 5 wt% to about 25 wt%, about 5 wt% to about 22 wt%, about 5 wt% to about 20 wt%, about 5 wt% to about 19 wt%, about 5 wt% to about 18 wt%, about 5 wt% to about 17 wt%, about 5 wt% to about 16 wt%, about 5 wt% to about 15 wt%, about 6 wt% to about 30 wt%, about 6 wt% to about 25 wt%, about 6 wt% to about 22 wt%, about 6 wt% to about 20 wt%, about 6 wt% to about 19 wt%, about 6 wt% to about 18 wt%, about 6 wt% to about 17 wt%, about 6 wt% to about 16 wt%, or about 6 wt% to about 15 wt%.

In an embodiment, the oil phase may be included, based on the total weight of the liquid crystal composition, in an amount in a range of about 5 wt% to about 40 wt%, for example, about 5 wt% to about 35 wt%, about 5 wt% to about 30 wt%, about 5 wt% to about 25 wt%, about 5 wt% to about 24 wt%, about 5 wt% to about 23 wt%, about 5 wt% to about 22 wt%, about 5 wt% to about 21 wt%, about 5 wt% to about 20 wt%, about 5 wt% to about 19 wt%, about 5 wt% to about 18 wt%, about 5 wt% to about 17 wt%, about 5 wt% to about 16 wt%, about 5 wt% to about 15 wt%, about 7 wt% to about 40 wt%, about 7 wt% to about 35 wt%, about 7 wt% to about 30 wt%, about 7 wt% to about 25 wt%, about 7 wt% to about 24 wt%, about 7 wt% to about 23 wt%, about 7 wt% to about 22 wt%, about 7 wt% to about 21 wt%, about 7 wt% to about 20 wt%, about 7 wt% to about 19 wt%, about 7 wt% to about 18 wt%, about 7 wt% to about 17 wt%, about 7 wt% to about 16 wt%, about 7 wt% to about 15 wt%, about 10 wt% to about 40 wt%, about 10 wt% to about 35 wt%, about 10 wt% to about 30 wt%, about 10 wt% to about 25 wt%, about 10 wt% to about 24 wt%, about 10 wt% to about 23 wt%, about 10 wt% to about 22 wt%, about 10 wt% to about 21 wt%, about 10 wt% to about 20 wt%, about 10 wt% to about 19 wt%, about 10 wt% to about 18 wt%, about 10 wt% to about 17 wt%, about 10 wt% to about 16 wt%, or about 10 wt% to about 15 wt%.

In an embodiment, the liquid crystal composition may include: a combination of Ceramide3 (Ceramide NP) and Ceramide 6 (Ceramide AP) (optionally, further including Ceramide 1 (Ceramide EOP), Ceramide 10 (Ceramide Nds), Ceramide 11 (Ceramide Ads), and phytosphinosine); as a surfactant, a combination of glyceryl monostearate, cetyl alcohol, and hydrogenated lecithin; and/or cholesterol (for example, in an amount in a range of about 0.1 wt% to about 2.0 wt%).

In an embodiment, the liquid crystal composition may have a hexagonal system crystal structure.

The term "hexagonal system" as used in the present specification is used interchangeably with "hexagonal structure", "hexagonal type", or "hexagonal crystal system", and may refer to a crystal structure in which substances such as surfactants in an emulsion are arranged. In addition, the hexagonal system shows sharp single peaks derived from the cross-section in the wide angle region. For example, in the hexagonal system, a peak is observed at 1.52 Å⁻¹ in wide-angle X-ray scattering analysis. Since the hexagonal system structure typically has a loose or open crystal structure, the polarization easily occurs due to increased molecular motion, and thus the hexagonal system structure has high piezoelectric properties that can generate microcurrents and has an effect of promoting skin absorption of physiologically active substances. Accordingly, the liquid crystal composition may be able to promote skin absorption (transdermal delivery) of ceramide or a derivative thereof or a physiologically active substance.

In an embodiment, the liquid crystal composition may be an emulsion formulation.

The liquid crystal composition may be any one formulation of an oil-in-water type, a water-in-oil type, and a non-aqueous dispersion. The term "oil-in-water (O/W)" is used interchangeably with "oil-in-water emulsion", and refers to a form in which oil is dispersed in water. The term "water-in-oil (W/O)" is used interchangeably with "water-in-oil emulsion", and refers to a form in which water is dispersed in oil. The term "non-aqueous dispersion" refers to an form in which dispersion not in water, but in a solvent.

In an embodiment, the liquid crystal composition may particularly have an O/W emulsion formulation. However, the formulation is not particularly limited thereto, and depending on a formulation to be prepared, mixing ingredients for a liquid crystal composition commonly used in the technical field to which the invention pertains may be included. The O/W emulsion formulation may be prepared according to a method known in the art by mixing water phase and oil phase ingredients used in the preparation of a general cosmetic emulsion.

In an embodiment, the liquid crystal composition may have an average size (diameter) in a range of about 1 µm to about 50 µm, about 1 µm to about 45 µm, about 1 µm to about 40 µm, about 1 µm to about 35 µm, about 1 µm to about 30 µm, about 5 µm to about 40 µm, about 5 µm to about 35 µm, or about 5 µm to about 35 µm.

The liquid crystal composition may have high liquid crystal stability in which the liquid crystals are maintained even after exposure to a high temperature (e.g., 45 °C) for 4 weeks according to the compositional ratio of Ceramide 3 (Ceramide NP), Ceramide 6 (Ceramide AP), and Ceramide 1 (Ceramide EOP).

In this regard, by including hybrid ceramides having high stability, the liquid crystal composition may have effects on the skin moisturizing and recovery of damaged skin.

Another aspect provides a method of preparing the liquid crystal composition, the method including: preparing an oil phase by mixing and dissolving a skin lipid that includes ceramide, cholesterol or a derivative thereof, and a fatty acid; preparing a water phase; and mixing the oil phase and the water phase.

Another aspect provides a cosmetic composition including: a liquid crystal composition including an aqueous phase and an oily phase including skin lipids, an emulsifier, and an oil, wherein the skin lipid comprises ceramide, cholesterol or a derivative thereof, and a fatty acid..

Another aspect provides a composition of external skin preparation including the liquid crystal composition.

In an embodiment, the liquid crystal composition, the cosmetic composition, or the composition of external skin preparation may be used for the amelioration of skin conditions.

In an embodiment, the amelioration of skin conditions include skin barrier strengthening, amelioration of skin aging, amelioration of skin wrinkles, amelioration of skin elasticity, skin regeneration, or skin whitening.

The term "skin aging" as used in the present specification may refer to tangible and intangible changes that appear in the skin with age. For example, such changes may include thinning of the epidermis and reduction in terms of the number of cells or blood vessels in the dermis, DNA damage repair ability, cell replacement cycle, wound healing, skin barrier function, moisture retention in epidermis, sweat secretion, sebum secretion, vitamin D production, physical injury defense, ability to remove chemicals, immune response, sensory function, and thermoregulation.

The skin aging may include not only premature aging symptoms induced by external stimuli, such as ultraviolet rays, pollution, cigarette smoke, and chemicals, but also natural aging phenomena caused by a decrease in the proliferation of skin cells with age, and may include all the wrinkles, reduced elasticity, sagging skin, and dryness. In addition, the wrinkles may include those induced by changing components constituting the skin by stimulation caused by changed in internal or external factors.

The aging may be photoaging. The term "photoaging" may be a phenomenon caused by external environmental factors, such as ultraviolet rays. Ultraviolet rays cause damage to biocomponents, such as activation of proteolytic enzymes, chain scission and abnormal cross-linking of matrix proteins, and repetition of these mechanisms can lead to visually apparent skin aging.

The term "wrinkles" as used in the present specification may refer to a state in which the skin elasticity is reduced. For example, the wrinkles may refer to skin folds. Pigmentation may refer to a case where the amount of pigments in the body is abnormal or a state having abnormality in a region where pigments appear. For example, pigmentation may include melasma, freckles, and the like. The term "prevention or amelioration of skin wrinkles" may refer to any action of preventing or ameliorating wrinkles by suppressing the expression of wrinkle-related factors or any action of increasing the total amount of collagen.

The "skin barrier strengthening" may refer to any action that enhances the function of the skin barrier that is located at the outermost layer of the skin and prevents loss of moisture and nutrients.

The "skin whitening" may brighten the skin tone by inhibiting the synthesis of melanin pigments, and may refer to amelioration of skin hyperpigmentation such as melasma and freckles caused by ultraviolet rays, hormones, or heredity.

The cosmetic composition may be prepared in any formulation commonly prepared in the art to which the present invention pertains. For example, the cosmetic composition may be formulated into a softening lotion, a nutrient lotion, an emulsion, a lotion, a cream, a paste, a gel, a mask pack, a solution, a suspension, an oil, a wax, a powder, a foundation, a spray, a hair product, a surfactant-containing cleansing, and the like, but the formulation is not limited thereto. More specifically, the cosmetic composition may be formulated into a skin lotion, a skin softener, a skin toner, a nourishing cream, a massage cream, a milk lotion, a powder, an essence, an eye cream, a sunscreen lotion, a sunscreen cream, a makeup primer, a makeup base, a BB cream, a powder-type foundation, an emulsion-type foundation, a cleansing cream, a cleansing foam, a cleansing water, a soap, a mask pack, a stick-type product, a balm-type product, a spray, or a powder.

The cosmetic composition may include additional ingredients commonly used in cosmetics, for example, any conventional cosmetic ingredient selected from a thickener, a dispersant, a fragrance, a filler, a preservative, an antiseptic, a neutralizer, a sweetener, a vitamin, a free-radical scavenger, a metal ion sequestering agent, a functional ingredient, and a mixture thereof. One skilled in the art may select any additional ingredients and/or amounts thereof such that the beneficial properties of the liquid crystal composition according to the present disclosure are not adversely affected or not substantially affected by the anticipated addition.

In an embodiment, the cosmetic composition may have one formulation selected from the group consisting of a toner, a lotion, a cream, an essence, a mask pack, a foundation, a soap, a cleansing product, a body moisturizer, a hair product, and a cleanser.

In an embodiment, the formulation of the cosmetic composition may further include at least one selected from a stabilizer, a surfactant, oil, a chelating agent, polyol, a preservative, and a thickener.

The cosmetic composition may further include additional ingredients commonly used in cosmetics. One skilled in the art may select any additional ingredients and/or amounts thereof such that the beneficial properties of the liquid crystal composition according to the present disclosure are not adversely affected or not substantially affected by the anticipated addition.

In the present specification, the external skin preparation may be a cream, gel, an ointment, a skin emulsifier, a skin suspension, a transdermal delivery patch, a drugcontaining bandage, a lotion, or a combination thereof. The external skin preparation may be, as needed, appropriately blended with an ingredient commonly used in external skin preparation for cosmetics or pharmaceuticals, such as a water-based ingredient, an oil-based ingredient, a powder ingredient, an alcohol-based ingredient, a moisturizer, a thickener, an ultraviolet absorber, a whitening agent, a preservative, an antioxidant, a surfactant, a fragrance, a colorant, various skin nutrients, or a combination thereof. The external skin preparation may include: a metal sequestering agent, such as disodium edetate, trisodium edetate, sodium citrate, sodium polyphosphate, sodium metaphosphate, gluconic acid, and the like: various natural medications, such as caffeine, tannin, verapamil, a licorice extract, glabridin, a hot-water extract of Chinese quince fruit, or the like: drugs, such as tocopherol acetate, glycyrrhizic acid, and tranexamic acid and a derivative or salt thereof, and the like, saccharides, saccharide, such as vitamin C, magnesium phosphate ascorbate, glucoside ascorbate, arbutin, koji acid, glucose, fructose, trehalose, and the like.

The cosmetic composition or external skin preparation may be used alone, applied repeatedly for use, or applied repeatedly with other cosmetic compositions other than the liquid crystal composition of the present disclosure for use. In addition, the cosmetic composition according to the present disclosure may be used according to a conventional usage method, and the number of times of use may vary according to the skin conditions or taste of a user.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is an image of a liquid crystal composition according to an embodiment observed with a polarizing microscope;
FIG. 2 is an image of a liquid crystal composition according to an embodiment for analyzing the stability by using a polarizing microscope;
FIG. 3 is an image (upper panel) and a graph (lower panel) that show effects of a liquid crystal composition according to an embodiment on the production amount of ceramide in the skin;
FIG. 4 is an image (upper panel) and a graph (lower panel) that show effects of a liquid crystal composition according to an embodiment on the expression level of aquaporin 3 in the skin;
FIG. 5 is an image (upper panel) and a graph (lower panel) that show effects of a liquid crystal composition according to an embodiment on the expression level of filaggrin in the skin;
FIG. 6 is a graph that shows effects of a liquid crystal composition according to an embodiment on the expression level of hyaluronic acid in the skin;
FIG. 7 is an image (upper panel) and a graph (lower panel) that show effects of a liquid crystal composition according to an embodiment on the expression level of aquaporin 3 in the undifferentiated skin;
FIG. 8 is an image (upper panel) and a graph (lower panel) that show effects of a liquid crystal composition according to an embodiment on the expression level of loricrin in the undifferentiated skin;
FIG. 9 is an image (upper panel) and a graph (lower panel) that show effects of a liquid crystal composition according to an embodiment on the expression level of involucrin in the undifferentiated skin; and
FIG. 10 is an image that shows effects of a liquid crystal composition according to an embodiment on the thickness of undifferentiated skin.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

Hereinafter, preferable Examples are presented to help understanding of the disclosure. However, the following examples are only presented for easier understanding of the disclosure, and the contents of the disclosure are not limited by the following examples. Various transformations can be applied in the examples, and thus examples are not limited to the following examples, and can be implemented in various forms.

### Preparation of liquid crystal composition, Examples 1 to 3 and Comparative Examples.

The ingredients listed in Table 1 below were mixed to prepare liquid crystal comparative Examples of Examples 1 to 3 and Comparative Examples 1 and 2.

Here, the amounts of the following ingredients all expressed in wt%.

In detail, to prepare a water phase, xanthan gum was dissolved in water at room temperature. Afterwards, glycerin and 1,2-hexanediol were added to the resultant solution, stirred, and heated at a temperature up to 75 °C to 80 °C, so as to prepare a water phase. To prepare an oil phase, an emulsifier (e.g., hydrogenated lecithin, glyceryl monostearate, and cetearyl alcohol), a skin lipid (e.g., cholesterol, stearic acid, ceramide), and an additional sphingolipid (e.g., glucosyl ceramide, tetraacetylphytosphingosine, phytosphingosine) were sequentially added to oil (e.g., hydrogenated polydecene), and completely dissolved at a temperature in a range of about 90 °C to about 100 °C. Next, while maintaining the temperature between 75 °C and 85 °C, the oil phase was stirred and introduced to the water phase. After the introduction of the oil phase was finished, homo-mixing (3000 rpm) was performed thereon for 5 minutes. Afterwards, by rapid cooling down to 35 °C to 40 °C, the liquid crystal composition was prepared.

**Table 1**

| Ingredients | | Example 1 | Example 2 | Example 3 | Compara tive Example 1 | Compara tive Example 2 |
|---|---|---|---|---|---|---|
| Oil phase | Hydrogenated polydecene | 7.00 | 10.00 | 10.00 | 7.00 | 10.00 |
| | Glyceryl monostearate | 3.00 | 3.50 | 3.50 | - | 3.00 |
| | Ceteareth-20 | - | - | - | 3.00 | - |
| | Cetyl alcohol | 3.00 | 2.00 | 2.00 | 3.00 | 3.00 |
| | Hydrogenated lecithin | 1.50 | 1.00 | 1.00 | 1.50 | 0.50 |
| | Ceramide NP | 0.50 | 1.00 | 1.00 | 0.50 | 1.00 |
| | Ceramide AP | 0.10 | 0.50 | 0.50 | 0.10 | 0.50 |
| | Ceramide EOP | - | 0.01 | 0.01 | - | 0.01 |
| | Ceramide NG | - | - | 0.10 | - | - |
| | Ceramide AG | - | - | 0.10 | - | - |
| | Cholesterol | 0.30 | 0.90 | 1.00 | 0.30 | 2.70 |
| | Stearic acid | 0.30 | 0.90 | 1.00 | 0.30 | 0.90 |
| | Phytosphingosine | - | 0.5 | - | - | 0.5 |
| | Tetraacetylphytosphingosin e | 0.25 | 0.25 | 0.25 | 0.25 | 0.25 |
| | Glucosylceramide | 0.001 | 0.001 | 0.001 | 0.001 | 0.001 |
| Water phase | Purified water | 77.049 | 72.439 | 72.539 | 77.049 | 70.639 |
| | Glycerin | 5.00 | 5.00 | 5.00 | 5.00 | 5.00 |
| | 1,2-hexanediol | 2.00 | 2.00 | 2.00 | 2.00 | 2.00 |
| | Total | 100 | 100 | 100 | 100 | 100 |

In the liquid crystal compositions of Examples 1 to 3, the mass ratio of ceramide:cholesterol:fatty acid was 40:25:25. However, the liquid crystal composition was prepared to include these ingredients at a ratio of 1:1:1 when the mass ratio was converted to a molar ratio.

### Experimental Example 1. Analysis of crystal structure of liquid crystal composition

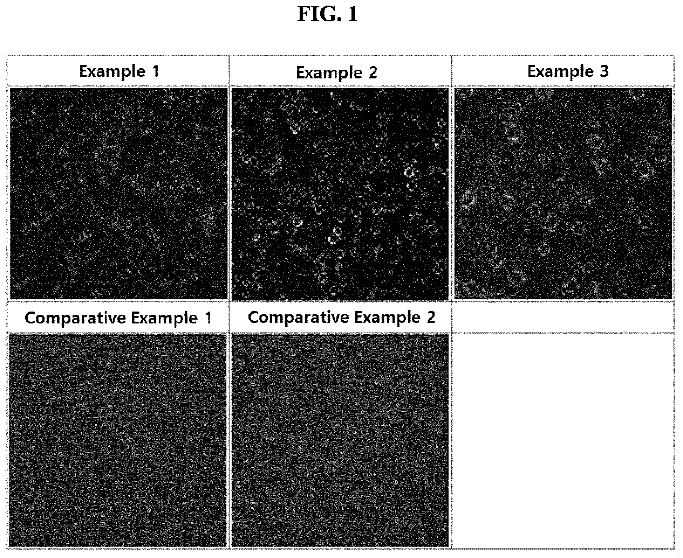

The crystals of the liquid crystal compositions of Examples 1 to 3 and Comparative Examples 1 to 2 were analyzed with a polarizing microscope, and the results are shown in FIG. 1.

As shown in FIG. 1, it was confirmed that the crystals of the liquid crystal compositions of Examples 1 to 3 showed a homogeneous hexagonal liquid crystal form well, whereas the crystals of the liquid crystal compositions of Comparative Examples 1 and 2 did not form hexagonal liquid crystals well.

### Experimental Example 2. Analysis of stability of liquid crystal composition

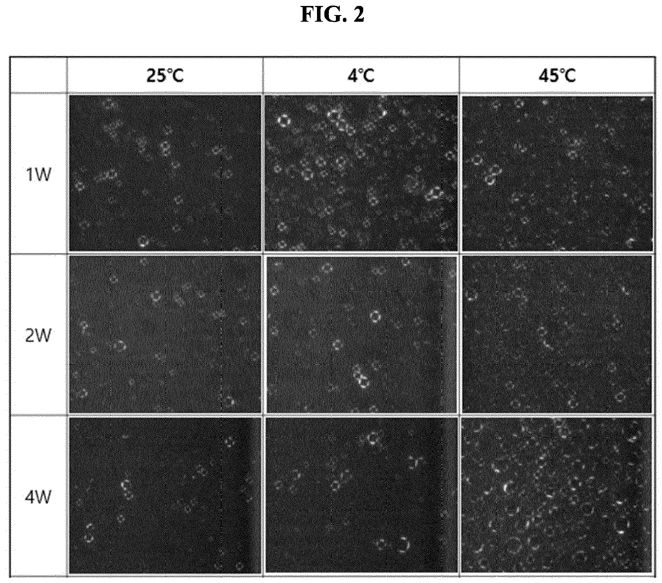

To analyze the stability of the liquid crystal composition, the liquid crystal composition of Example 2 was stored at different temperatures (25 °C, 4 °C, and 45 °C), and then the presence or absence of liquid crystals was analyzed with a polarizing microscope every week, and the results are shown in FIG. 2.

As shown in FIG. 2, it was confirmed that the liquid crystals of the liquid crystal composition according to Example was maintained for 4 weeks at all temperatures (25 °C, 4 °C, and 45 °C).

### Experimental Example 3. Confirmation of ceramide production amount using artificial skin

To confirm the amount of ceramide produced by the liquid crystal composition, the liquid crystal compositions of Examples 1 and 2 were applied to artificial skin (EPI-200-X, MatTek), and after 4 hours, the permeation and production amount of ceramide were confirmed. The amount of ceramide was analyzed by immunofluorescence. Specifically, artificial skin was treated with the liquid crystal compositions of Examples, cultured for 24 hours, and then washed out by using PBS. The resultant artificial skin was put into a cassette and fixed with 10 % formalin. After cutting the artificial skin tissue to a thickness of 3 µm to prepare a slide, paraffin was melt thereon in a dry oven at a temperature of 60 °C for 30 minutes. To remove the paraffin, each slide was treated with xylene for 15 minutes, 100 % ethanol for 5 minutes, 95 % ethanol for 5 minutes, and 70 % ethanol for 5 minutes, respectively, and then subjected to cement hydration with running water. Antigen retrieval was performed on the resultant slides by heating in a citrate buffer for 10 minutes by using a cooker device, followed by cooling in running water. After treated with 0.5 % triton X-100 for 15 minutes, the resultant slides were washed with PBS buffer three times for 5 minutes each. Blocking with 1 % BSA was then performed thereon, followed by washing with PBS three times for 5 minutes each.

The primary antibody was diluted at 1:50 and reacted overnight at 4 °C, and citrate buffer was used for antigen retrieval.

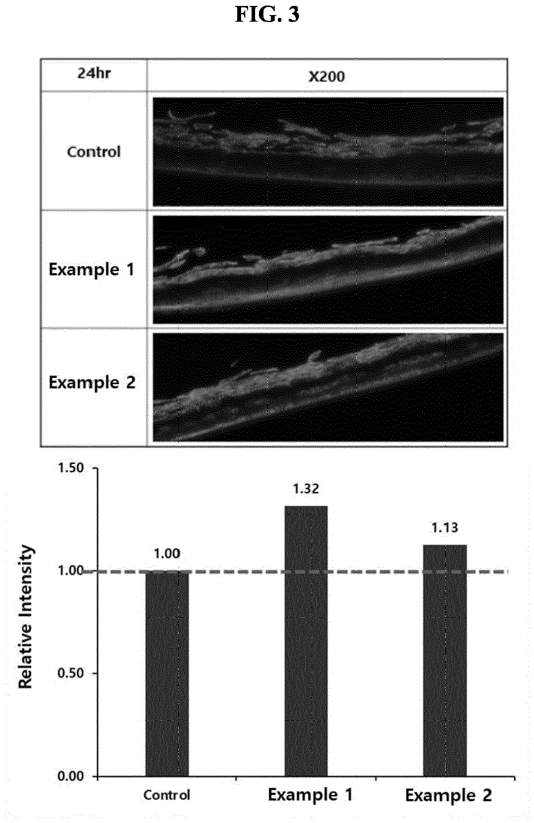

After washing with PBS, the FITC 488 antibody was reacted for 1 hour at room temperature and washed three times with PBS. The slides were fixed by DAPI staining and using a vectashield mounting agent, and then observed under a fluorescence microscope. As a control group, an untreated liquid crystal composition was used. The analysis results are shown in FIG. 3.

As shown in FIG. 3, it was confirmed that the amount of ceramide produced in the skin was increased according to the application of the liquid crystal composition of Examples.

### Experimental Example 4. Confirmation of expression level of aquaporin 3 by using artificial skin

To confirm the change in the expression level of aquaporin 3 by the liquid crystal composition, the liquid crystal compositions of Examples 1 and 2 were applied to artificial skin (EPI-200-X, MatTek), and after 24 hours, the expression of aquaporin 3 was analyzed in the same manner as in Experimental Example 3 according to immunofluorescence.

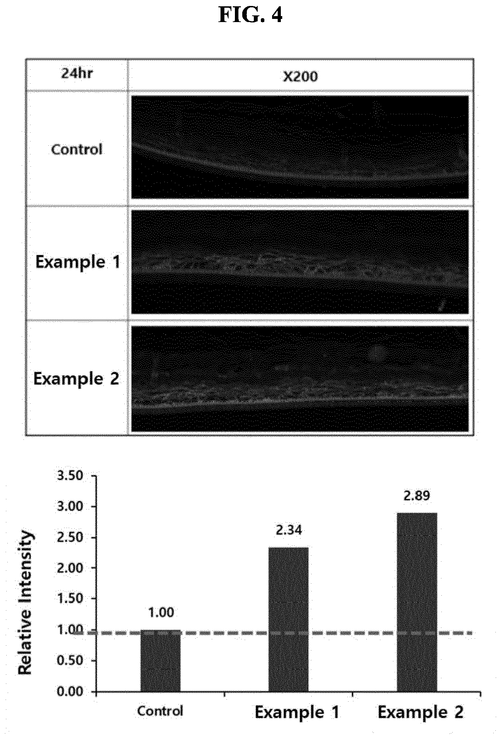

As shown in FIG. 4, it was confirmed that the expression level of aquaporin 3 in the skin was increased according to the application of the liquid crystal composition of Examples.

### Experimental Example 5. Confirmation of expression level of filaggrin by using artificial skin

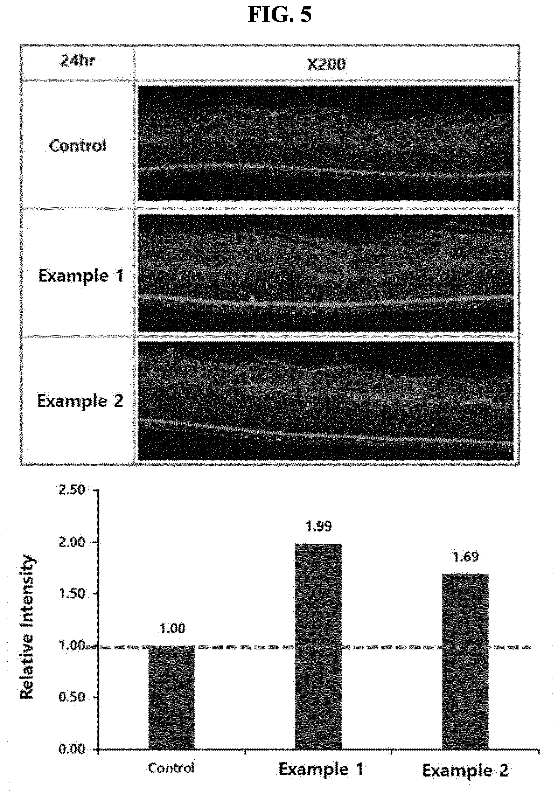

To confirm the change in the expression level of filaggrin by the liquid crystal composition, the liquid crystal compositions of Examples 1 and 2 were applied to artificial skin (EPI-200-X, MatTek), and after 24 hours, the expression of filaggrin was analyzed in the same manner as in Experimental Example 3 according to immunofluorescence. The results are shown in FIG. 5.

As shown in FIG. 5, it was confirmed that the expression level of filaggrin in the skin was increased according to the application of the liquid crystal composition of Examples.

### Experimental Example 6. Confirmation of hyaluronic acid production amount using artificial skin

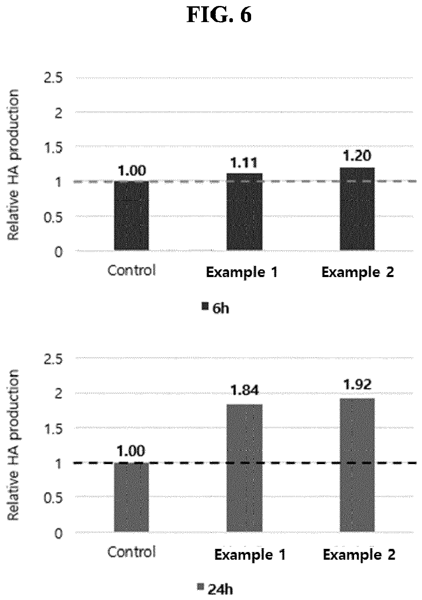

To confirm the amount of hyaluronic acid produced by the liquid crystal composition, the liquid crystal compositions of Examples 1 and 2 were applied to artificial skin (EPI-200-X, MatTek), and after 6 hours, the amount of hyaluronic acid produced was confirmed. The amount of hyaluronic acid was analyzed by using a Hyaluronan DuoSet ELISA (DY3614-05) according to the instructions of the manufacturer, and the results are shown in FIG. 6.

As shown in FIG. 6, it was confirmed that, according to the application of the liquid crystal compositions of Examples, the amount of hyaluronic acid produced after 6 hours was increased compared to the control group, and that the amount of hyaluronic acid produced after 24 hours was significantly increased compared to the control group.

Based on the results above, it was confirmed that the liquid crystal compositions of Examples had effects on the amelioration of the skin conditions, for example, strengthening the skin barrier and moisturizing the skin.

### Experimental Example 7. Confirmation of expression level of aquaporin 3 by liquid crystal composition

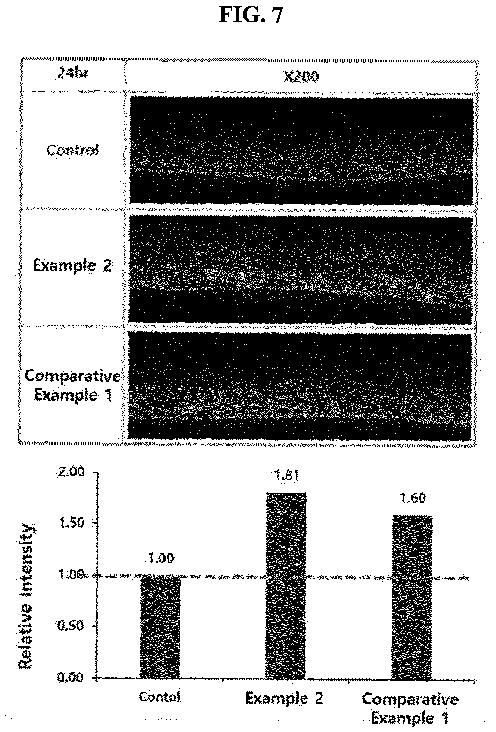

To confirm the change in the skin moisturizing ability, specifically the expression level of aquaporin 3, by the liquid crystal composition, the liquid crystal compositions of Example 2 and Comparative Example 1 were applied to undifferentiated artificial skin (EPI-201, MatTek), and after 24 hours, the expression level of aquaporin 3 was analyzed in the same manner as in Experimental Example 3 according to immunofluorescence. The results are shown in FIG. 7.

As shown in FIG. 7, as the skin differentiation process proceeded, the expression level of aquaporin 3 was increased regardless of the presence or absence of the liquid crystal composition of Example. However, it was confirmed that the expression level of aquaporin 3 in Example 2 was significantly improved compared to Comparative Example 1 having a non-liquid crystal structure.

### Experimental Example 8. Confirmation of expression level of loricrin by liquid crystal composition

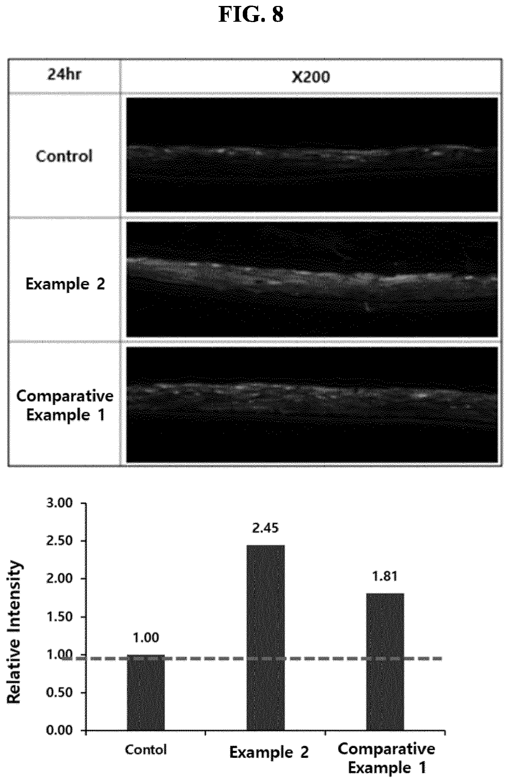

To confirm the change in the skin differentiation factors, specifically the expression level of loricrin, by the liquid crystal composition, the liquid crystal compositions of Example 2 and Comparative Example 1 were applied to undifferentiated artificial skin (EPI-201, MatTek), and after 24 hours, the expression level of loricrin was analyzed in the same manner as in Experimental Example 3 according to immunofluorescence. The results are shown in FIG. 8.

As shown in FIG. 8, it was confirmed that the expression level of loricrin in the skin was increased according to the application of the liquid crystal composition of Examples. In addition, it was confirmed that the expression level of loricrin in the liquid crystal structure of Example was significantly greater than the expression level of loricrin in the non-liquid crystal structure of Comparative Example.

### Experimental Example 9. Confirmation of expression level of involucrin by liquid crystal composition

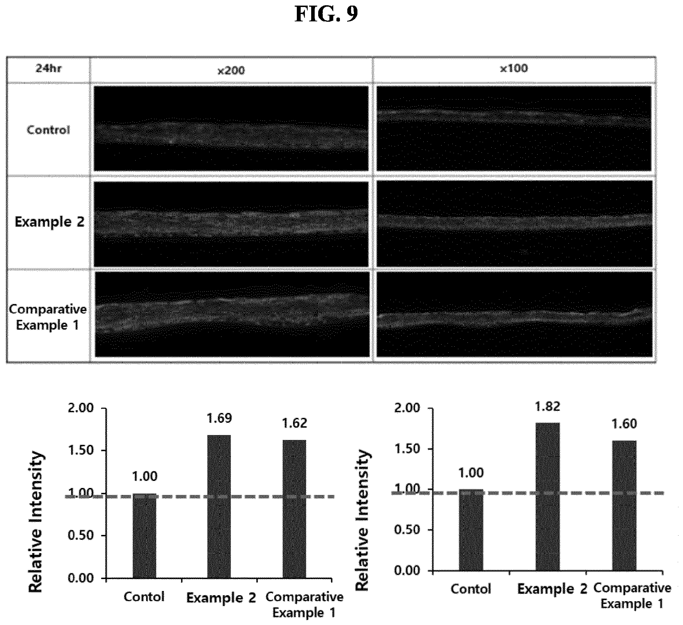

To confirm the change in the skin differentiation factors, specifically the expression level of involucrin, by the liquid crystal composition, the liquid crystal compositions of Example 2 and Comparative Example 1 were applied to undifferentiated artificial skin (EPI-201, MatTek), and after 24 hours, the expression level of involucrin was analyzed in the same manner as in Experimental Example 3 according to immunofluorescence. The results are shown in FIG. 9.

As shown in FIG. 9, it was confirmed that the expression level of involucrin in the skin was increased according to the application of the liquid crystal composition of Examples. In addition, it was confirmed that the expression level of involucrin in the liquid crystal structure of Example was significantly greater than the expression level of involucrin in the non-liquid crystal structure of Comparative Example.

### Experimental Example 10. Confirmation of skin thickness and differentiation degree by liquid crystal composition

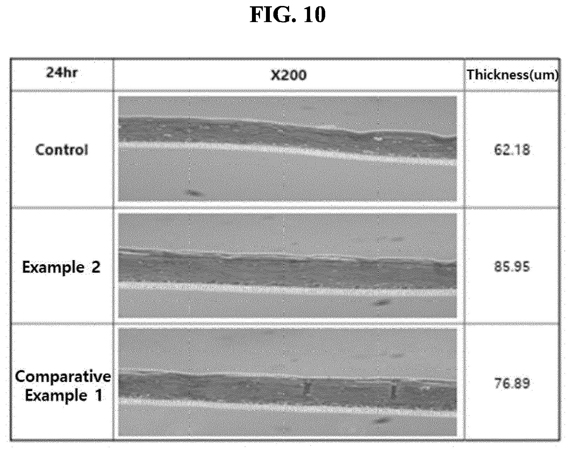

To confirm the difference in skin differentiation and skin barrier thickness by the liquid crystal composition, the liquid crystal compositions of Example 2 and Comparative Example 1 were applied to undifferentiated artificial skin (EPI-201, MatTek), and after 24 hours, the skin thickness was measured. The skin thickness was confirmed through H&E staining. In detail, in the same manner as in Experimental Example 3,a paraffin block was prepared, and a slide was prepared by cutting the artificial skin tissue to a thickness of 3 µm to remove paraffin, and then paraffin was melt thereon in a dry oven at a temperature of 60 °C for 30 minutes. The slide was immersed in xylene, 100 % ethanol, 85 % ethanol, 70 % ethanol, and water in this stated order, followed by staining with hematoxylin for 10 minutes. After washing with running water, the resultant slide was immersed in 1 % HCI and 1 % ammonia in this stated order. After staining with eosin for 2 minutes, dehydration was performed thereon, and the resultant slide was mounted, and the results obtained by observation under a microscope are shown in FIG. 10.

As shown in FIG. 10, it was confirmed that the skin thickness and differentiation of skin cells were increased according to the application of the liquid crystal composition of Examples. In addition, it was confirmed that the skin thickness in the liquid crystal structure of Example was significantly greater than the skin thickness in the non-liquid crystal structure of Comparative Example.

Based on the results above, it was confirmed that the liquid crystal compositions of Examples had effects of improving the condition of undifferentiated skin, for example, strengthening skin barrier and inducing skin differentiation.

According to a liquid crystal composition including various types of ceramide of an aspect and a method of preparing the same, the liquid crystal composition not only has high liquid crystal stability that liquid crystal is maintained even after 4 weeks at a high temperature, but also activities of increasing an amount of ceramide, increasing an expression level of aquaporin 3, increasing an amount of filaggrin, increasing an expression level of hyaluronic acid, increasing an expression level of loricrin, increasing an expression level of involucrin, and increasing a thickness of the skin, resulting in effects usefully available for amelioration of the skin conditions.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope as defined by the following claims.

## Claims

1. Liquid crystal composition comprising:
a water phase; and
an oil phase comprising a skin lipid, an emulsifier, and oil,
wherein the skin lipid comprises ceramide, cholesterol or a derivative thereof, and a fatty acid.

2. The liquid crystal composition of claim 1, wherein the skin lipid comprises in an amount in a range of about 0.1 wt% to about 10 wt% based on the total weight of the composition.

3. The liquid crystal composition of claim 1, wherein the emulsifier comprises in an amount in a range of about 1 wt% to about 20 wt% based on the total weight of the composition.

4. The liquid crystal composition of claim 1, wherein the oil is comprised in an amount in a range of about 5 wt% to about 30 wt% based on the total weight of the composition.

5. The liquid crystal composition of claim 1, wherein the skin lipid comprises the ceramide, the cholesterol or a derivative thereof, and the fatty acid at a molar ratio of 1: 0.5 to 2: 0.5 to 2.

6. The liquid crystal composition of claim 1, wherein the ceramide comprises natural ceramide, synthetic ceramide, or pseudo ceramide.

7. The liquid crystal composition of claim 1, wherein the ceramide comprises any one selected from the group consisting of Ceramide 1 (Ceramide EOP), Ceramide 2 (Ceramide NS), Ceramide 3 (Ceramide NP), Ceramide 4 (Ceramide EOH), Ceramide 5 (Ceramide AS), Ceramide 6 (Ceramide AP), Ceramide 7 (Ceramide AH), Ceramide 8 (Ceramide NH), Ceramide 9 (Ceramide EOS), Ceramide 10 (Ceramide Nds), Ceramide 11 (Ceramide Ads), Ceramide 12 (Ceramide EOds), and a combination thereof.

8. The liquid crystal composition of claim 1, wherein the ceramide is hybrid ceramides in which Ceramide3 (Ceramide NP) and Ceramide 6 (Ceramide AP) are mixed at a weight ratio in a range of about 5:1 to about 1:1.

9. The liquid crystal composition of claim 8, wherein the ceramide is hybrid ceramides further comprising Ceramide 9 (Ceramide EOS).

10. The liquid crystal composition of claim 8, wherein the ceramide is hybrid ceramides further comprising two or more ceramides selected from Ceramide 9 (Ceramide EOS), Ceramide 10 (Ceramide Nds), and Ceramide 11 (Ceramide Ads).

11. The liquid crystal composition of claim 1, further comprising any one selected from sphingosine, phytosphingosine, tetraacetylphytosphingosine, and spinganine.

12. The liquid crystal composition of claim 1, the emulsifier has hydrophilelipophile balance (HLB) in a range of about 3 to about 9.

13. The liquid crystal composition of claim 1, wherein the liquid crystal composition has an emulsion formulation.

14. The liquid crystal composition of claim 1, wherein the liquid crystal composition is for ameliorating skin conditions.

15. A cosmetic composition comprising the liquid crystal composition of any one of claims 1 to 14.
